# EUROPEAN PATENT SPECIFICATION

(11) **EP 2 459 117 B1**
(45) Date of publication and mention of the grant of the patent: **29.05.2019**
(21) Application number: 10771314.1
(22) Date of filing: 31.07.2010
(51) Int. Cl.: A61F 2/00, A61M 25/04, A61M 25/10

(54) **CATHETER FOR TRANSANAL IRRIGATION**
KATHETER FÜR TRANSANALE SPÜLUNG
CATHÉTER POUR IRRIGATION TRANSANALE

(30) Priority: 31.07.2009 FR 0903818
(43) Date of publication of application: 06.06.2012
(73) Proprietor: B. Braun Medical Sas, 92210 Saint-Cloud (FR)
(72) Inventor: BIDAULT, Guy, 28400 Nogent-le-Rotrou (FR); COLLIN, Rémi, 61340 Saint-Hilaire-sur-Erre (FR)
(74) Representative: August Debouzy
(86) International application number: PCT/EP2010/004691
(87) International publication number: WO 2011/012323

(56) References cited:
- EP-A1- 0 109 897
- WO-A1-02/26293
- WO-A1-2006/010556
- GB-A- 2 243 553
- JP-A- 10 179 750
- JP-A- 10 234 854
- US-A- 4 637 814
- US-A1- 2003 073 974

## Description

The present invention relates to a catheter for transanal irrigation.

Transanal irrigations are carried out, for example, before digestive surgical interventions on the bowel to clean and fully empty the intestine. Transanal irrigations may also be used to empty the intestine in a controlled manner in case of constipation or anal incontinence, that is, more generally, for the treatment of functional disorders of the large intestine. To do this, a catheter is introduced into the anus and water is injected into the colon at a temperature close to that of the body. For an adult patient, a volume of more than a litre can be injected. Devices to carry out transanal irrigation are for example known from EP 0 109 897 A1 and US 2003/0073974 A1. A rectal block device for patients suffering from faecal incontinence is known from GB 2 243 553 A.

During instillation of the irrigating liquid, the problem of leaks of this liquid through the anus arises. In fact, faecal continence is ensured by the anal sphincter in a healthy subject. More specifically, faecal continence is ensured by the external and, most importantly, internal sphincters, both of which spontaneously enter a state of relatively strong contraction and produce a pressure in the anal canal of around 40 mm Hg. This pressure is sufficient to prevent any involuntary leak of faecal matter.

In contrast, the resting pressure within the ampulla recti is normally very low, in the order of 2 to 3 mm Hg only. In the case of a sudden increase in intra-rectal pressure, a first recto-anal reflex causes a relaxing of the internal anal sphincter and a second recto-anal reflex, at the same time, produces a contraction of the external anal sphincter in order to prevent any involuntary leak of faecal matter.

The introduction into the anal canal of a device for administering irrigation liquid is sufficient to compromise anal continence by the simple fact of being an inert object foreign to the functioning of the sphincter. Moreover, transanal irrigation is used for patients for whom the normal functioning of the colon, rectum and anus and, consequently, often also anal continence, is disrupted. The use of a simple tube or simple catheter for the administration of irrigation liquid would therefore not be sufficient, since it would lead to massive leaks of liquid and a very poor level of comfort during use.

Devices for the transanal injection of liquids have been known for a long time, e.g. the practice of injecting enema liquids into the rectum by means of giant syringes known by the name of "clysters", for which a great number of variants existed. More recently (1970), and in a slightly different context, proposals were made, for example, for a catheter for the transanal injection of contrast liquid with a rectal balloon catheter (patent US 3,509,884). This catheter is introduced through the anal canal into the rectum, when the balloon is inflated with air using a syringe. Two small additional balloons, placed facing one another on the catheter, at 2 or 3 cm below the main balloon, are inflated at the same time as the internal balloon and provide the operator with a marker so that the catheter is not introduced too deeply. However, the injection of a contrast liquid into the intestine is an isolated operation and implemented by caregiver personnel capable of handling the catheter and the contrast liquid in such a way as to minimise leaks and to manage those that are produced without difficulty. Transanal irrigation, however, is an operation that must, for the most part, be carried out regularly, over the long term and by the patient himself, at the rate of 2 to 3 irrigations per week. Moreover, very often these patients, due to a neurological deficiency (medullary lesion, multiple sclerosis), have reduced manual dexterity and it is essential to provide them with an instillation procedure that is as simple as possible and to maximally avoid leakage during the phase of instillation of the irrigation liquid. An intra-rectal balloon alone seems insufficient to ensure this watertight effect.

More recently, and in yet another context (collection of liquid stools from patients suffering from chronic anal incontinence), devices have been developed in which the watertight effect is obtained by means of a double balloon system where a first balloon chamber is found on the part of the catheter that is introduced into the rectum and where a second balloon chamber is positioned relative to the first in such a way that it is located outside the anus, in order to limit the depth of introduction of the catheter. A device of this type is described, for example, in document WO 2008/103788 A1. The disadvantage of this type of device is the fact that the diameter of the stool evacuation tube is necessarily quite large in order to avoid it becoming blocked during aspiration of the stools. For this reason, the positioning of this device is difficult and, to make it easier, the use of a special insertion accessory is recommended.

Taking into account the patient population for which the transanal irrigation catheter according to the invention is intended, the use of a special insertion device is not possible. Moreover, the diameter of the catheter according to the invention should be as small as possible in order to render the introduction easy and the wearing, during the phase of instillation of the irrigation liquid, as comfortable as possible.

The aim of the present invention is to create a catheter for transanal irrigation that has a sufficient watertight effect for a transanal irrigation with a significant quantity of liquid while also offering superior comfort to the patient.

This aim is achieved, in accordance with the invention, with a catheter, as claimed in claim 1, bearing the characteristics described below.

In accordance with the invention, the catheter for transanal irrigation comprises:
- a distal section that is sufficiently rigid to be introduced into the rectum of a patient, and which is provided with a rounded tip;
- a proximal section, intended to be connected to a source of irrigation liquid;
- a channel for conducting the irrigation liquid that opens out through at least one opening close to the distal tip of the distal section.

The distal section of the catheter according to the invention is provided with an inflatable distal balloon and an inflatable proximal balloon, both of which are intended to be positioned inside the rectum of a patient. The catheter is characterized in that the interior of the two balloons are in air communication and in that the distal balloon (or rectal balloon) has a greater extensibility and therefore, at equal pressure, a larger diameter than the proximal balloon (or anal balloon). The catheter, moreover, has on the proximal extremity of its distal section, close to the junction with the proximal section, a mark that makes it easier to introduce the catheter and indicates to the patient the maximum extent of introduction of the catheter into the rectum. This is particularly important for paraplegic patients, who no longer have any sensation in the anus area, and who could not otherwise determine the correct depth of introduction of the catheter.

The catheter according to the invention shows, compared to the state of the art, the particularity that the two inflatable balloons are located on the part of the catheter to be introduced into the rectum (anal canal and ampulla recti). In the case of the known catheters, described above, the catheter is simply retained in the ampulla recti by means of the distal balloon, the two external balloons serving only as a guide to prevent a too-deep introduction; or it is fixed by the two balloons, of which one is placed within the rectum and the other outside the anus, the catheter being in this manner fixed around the anal sphincter.

The interior configuration and the physiological or physiopathological functioning of the rectum is suitable to accommodate two balloons with different extensibilities and, at equal pressure, different sizes. In fact, to prevent leaks of irrigation liquid through the anus during the instillation of liquid into the rectum, the junction between the catheter and the wall of the rectum and sphincter (anal canal) must be rendered as watertight as possible. This watertightness effect is obtained, according to the invention, by means of a second balloon that is also located on the distal section of the catheter to be placed within the rectum. This second balloon is found in a proximal position relative to the first balloon and therefore in the terminal zone of the rectum, that is, in the anal canal and it has, preferably, a lower extensibility than that of the distal balloon.

The two balloons are in air communication with one another. This enables the inflating air to inflate, during the phase of instillation of the irrigation liquid, each of the two balloons exactly in accordance with the changes of pressure in the two respective regions of the rectum (ampulla recti and anal canal) and therefore to obtain an optimal junction between the walls, in particular of the anal canal, and the surface of the proximal balloon, without, moreover, distending the anal canal, since the inflation pressure of the two balloons is still less than the closure pressure of the anal canal. In particular, during instillation of the irrigation liquid, the increase of pressure in the ampulla recti may cause, by reflex, a relaxing of the internal anal sphincter and the communication between the two balloons then makes it possible for the air in the distal balloon to move towards the proximal balloon, which therefore increases slightly in volume (as the internal sphincter distends) and can therefore maintain a tight seal with the wall of the anal canal.

Preferably, the proximal section of the catheter is at least partially flexible. This allows for a greater comfort of use. The patient can introduce the catheter in the position most comfortable for him and bend the accessible proximal section of
the catheter such that the source of irrigation liquid may be easily connected with it. The proximal section of the catheter is in continuity with the distal section, but can be connected to it by means of a junction that is either straight, or angular, for example to make the catheter easier to use in the seated position on a toilet.

In order to inflate the balloons, a manual air blower could be provided. Any other means for inflating the balloons could also be used. The pressure necessary for inflating the balloons is regulated such that the two balloons show the volume and shape necessary to meet the objectives described above.

The distal section of the catheter according to the invention is, preferably, covered in a lubricating gel and provided with a protective covering made from a film of plastic. This covering preferably opens at the distal extremity and just before the introduction of the catheter, and it can be either folded in the direction of the proximal extremity during its introduction into the rectum, or removed entirely. Thus, the catheter may be delivered wrapped and ready for use. The patient no longer needs to cover the catheter with a lubricating gel, but simply to open the wrapping, and may use the catheter immediately. Preferably, the catheter is sterile.

One embodiment of the invention is illustrated in more detail below, with the help of the figures attached:
- **Figure 1**: shows a schematic plan view of an anal catheter according to the invention;
- **Figure 2**: shows a transversal view of an anal catheter according to the invention, in a position of introduction.

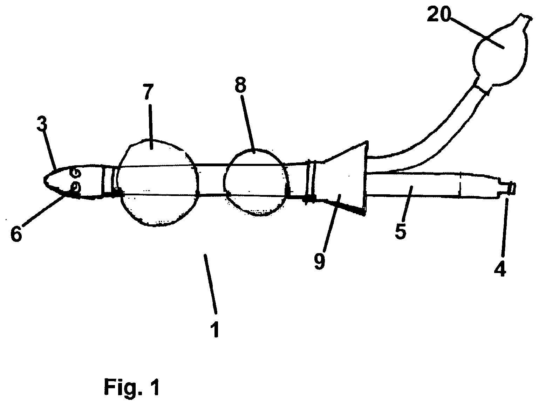

**Figure 1** shows a schematic plan view of a catheter for transanal irrigation according to the invention. The catheter comprises a distal section **1** and a proximal section **2**. The distal section **1** is sufficiently rigid to be introduced into the rectum of a patient. It is provided with a rounded tip **3**. The proximal section is provided with a connector **4** for connection to a source of irrigation liquid. The catheter has, moreover, a conduit **5** for an irrigation liquid, said conduit opening out into at least one opening **6** close to the rounded tip **3** of the distal section **1**.

The distal section of the catheter is provided with two inflatable balloons **7**, **8** intended to be positioned inside the rectum. The balloons can be inflated by means of a manual pump **20**.

In the figure, the balloons **7**, **8** are represented in an inflated state. Preferably, they are inflated simultaneously.

Moreover, it should be noted that the distal section **1** of the catheter has on its proximal extremity where said distal section is connected to the proximal section **2**, a conical collar **9**. This collar **9** shows the patient the depth to which he should introduce the catheter into the rectum. The balloons of the catheter can then be inflated and the catheter fixed in this position.

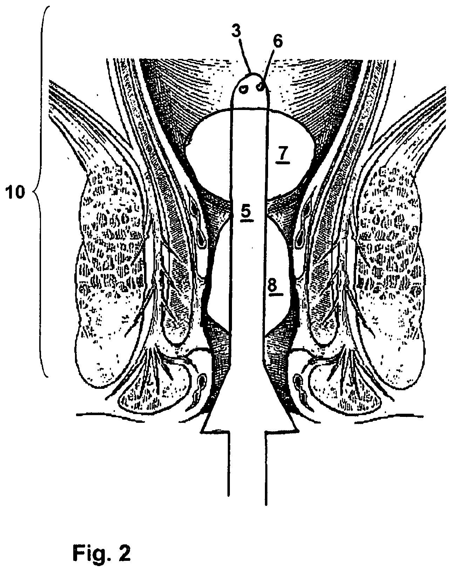

**Figure 2** shows a catheter of the type of figure **1** in longitudinal section. The distal section **1** is shown, which has a rounded tip **3** and is made sufficiently rigid to be introduced into the rectum **10** of a patient. The rounded tip **3** has an exit opening **6** connected via a conduit **4** with a source of irrigation liquid (not shown). The distal section **1** is provided, moreover, with two balloons **7**, **8** in communication with each other and inflated simultaneously. The catheter is introduced into the rectum of a patient with the balloons not inflated, then the balloons are inflated. Due to the communication between the two balloons, the inflating air is distributed between the two balloons such that their respective volumes and shapes are optimally suited to the configuration of the rectum and therefore produce an optimal watertight effect, but without generating a sensation of discomfort for the patient.

The invention is described above by way of example. It is understood, however, that the person skilled in the art is capable of carrying out different variations of the catheter for transanal irrigation without departing from the scope of the invention.

## Claims

1. Catheter for transanal irrigation comprising:
- a distal section (1) sufficiently rigid to be introduced into the rectum of a patient and provided with a rounded tip (3),
- a proximal section (2) intended to be connected to a source of irrigation liquid,
- a conduit channel (5) for the irrigation liquid, opening out through at least one opening (6) close to the distal tip (3) of the distal section (1),
wherein the distal section (1) of the catheter is provided with an inflatable distal balloon (7) and an inflatable proximal balloon (8) intended to be positioned inside the rectum
and the distal section (1) of the catheter has a mark (9) close to the junction with the proximal section
**characterised in that**
the interiors of the two balloons are in air communication, and
**in that** the distal balloon (7) has a greater extensibility than the proximal balloon (8).

2. Catheter according to claim 1, **characterised in that** the proximal section (2) is at least partly flexible.

3. Catheter according to claim 1 or 2, **characterised in that** the two balloons (7, 8) are inflatable simultaneously.

4. Catheter according to one of claims 1 to 3, **characterised in that** the mark on the distal section (1) takes the form of an annular bulge or of a collar (9).

5. Catheter according to one of claims 1 to 4, **characterised in that** the surface of the distal section (1) is covered with a lubricating gel and protected by a sleeve of watertight plastic film that can be folded back after opening.

6. Catheter according to one of claims 1 to 4, **characterised in that** the surface of the distal section (1) is covered with a lubricating gel and protected by a sleeve of watertight and removable plastic film.

## Patentansprüche

1. Katheter für eine transanale Spülung, umfassend:
- einen distalen Abschnitt (1), der hart genug ist, um das Rektum eines Patienten eingeführt zu werden, und mit einer abgerundeten Spitze (3) versehen ist,
- einen proximalen Abschnitt (2), der zum Anschließen an eine Spülflüssigkeitsquelle bestimmt ist,
- einen Kanal (5) für die Spülflüssigkeit, die über mindestens eine Öffnung (6) in der Nähe der distalen Spitze (3) des distalen Abschnitts (1) geöffnet ist,
wobei der distale Abschnitt (1) des Katheters mit einem aufblasbaren distalen Ballon (7) und einem aufblasbaren proximalen Ballon (8) versehen ist, die im Inneren des Rektums positioniert werden sollen,
und der distale Abschnitt (1) des Katheters in der Nähe der Verbindungsstelle mit dem proximalen Abschnitt eine Markierung (9) aufweist,
**dadurch gekennzeichnet, dass**
die Innenseiten der beiden Ballons miteinander in Luftverbindung stehen und
dass der distale Ballon (7) eine höhere Dehnbarkeit aufweist als der proximale Ballon (8).

2. Katheter nach Anspruch 1, **dadurch gekennzeichnet, dass** der proximale Abschnitt (2) mindestens teilweise flexibel ist.

3. Katheter nach Anspruch 1 oder 2, **dadurch gekennzeichnet, dass** die beiden Ballons (7, 8) gleichzeitig aufgeblasen werden können.

4. Katheter nach einem der Ansprüche 1 - 3, **dadurch gekennzeichnet, dass** die Markierung am distalen Abschnitt (1) in Form einer ringförmigen Ausbuchtung oder einer Manschette (9) vorliegt.

5. Katheter nach einem der Ansprüche 1 - 4, **dadurch gekennzeichnet, dass** die Oberfläche des distalen Abschnitts (1) mit einem Schmiergel bedeckt ist und von einer Hülse aus einer wasserdichten Kunststofffolie geschützt wird, die nach dem Öffnen zurückgefaltet werden kann.

6. Katheter nach einem der Ansprüche 1 - 4, **dadurch gekennzeichnet, dass** die Oberfläche des distalen Abschnitts (1) mit einem Schmiergel bedeckt ist und von einer Hülse aus einer abnehmbaren wasserdichten Kunststofffolie geschützt wird.

## Revendications

1. Cathéter pour irrigation transanale comprenant :
- une section distale (1) suffisamment rigide pour être introduite dans le rectum d'un patient, et munie d'une extrémité arrondie (3),
- une section proximale (2) destinée à être connectée à une source de liquide d'irrigation,
- un canal de conduite (5) du liquide d'irrigation qui s'ouvre dans au moins une ouverture (6) proche de l'extrémité distale (3) de la section distale (1),
dans lequel la section distale (1) du cathéter est munie d'un ballonnet distal gonflable (7) et d'un ballonnet proximal gonflable (8) destinés à être positionnés à l'intérieur du rectum,
et la section distale (1) du cathéter possède un repère (9) proche de la jonction avec la section proximale,
**caractérisé en ce que**
les intérieurs des deux ballonnets sont en communication par air, et
**en ce que** le ballonnet distal (7) possède une plus grande extensibilité que le ballonnet proximal (8).

2. Cathéter selon la revendication 1, **caractérisé en ce que** la section proximale (2) est au moins partiellement flexible.

3. Cathéter selon la revendication 1 ou 2, **caractérisé en ce que** les deux ballonnets (7, 8) sont gonflables en même temps.

4. Cathéter selon l'une des revendications 1 à 3, **caractérisé en ce que** le repère sur la section distale (1) prend la forme d'un renflement annulaire ou d'une collerette (9).

5. Cathéter selon l'une des revendications 1 à 4, **caractérisé en ce que** la surface de la section distale (1) est recouverte avec un gel lubrifiant et protégée par un manchon de film plastique étanche à l'eau qui peut être replié après ouverture.

6. Cathéter selon l'une des revendications 1 à 4, **caractérisé en ce que** la surface de la section distale (1) est recouverte avec un gel lubrifiant et protégée par un manchon de film plastique étanche à l'eau et amovible.
